# EUROPEAN PATENT APPLICATION

(11) **EP 2 554 213 A1**
(43) Date of publication of application: **06.02.2013**
(21) Application number: 11380064.3
(22) Date of filing: 01.08.2011
(51) Int. Cl.: A61M 39/02

(54) **Intelligent subcutaneous venous access port and method for detecting biolayer**

(71) Applicant: Centro de Estudios e Investigaciones Tecnicas (CEIT), 20018 San Sebastian Guipuzcoa (ES)
(72) Inventor: Paredes Puente, Jacobo, 20018 San Sebastian (Guipuzcoa) (ES); Arana Alonso, Sergio, 20304 Irún (Guipuzcoa) (ES); Arizti Urquijo, Fernando Jose, 20013 San Sebastian (Guipuzcoa) (ES); Schmidt, Lutz Christoph, 20018 San Sebastian (Guipuzcoa) (ES); Valderas Gazquez, Daniel, 20010 San Sebastian (Guipuzcoa) (ES)
(74) Representative: Cabinet Plasseraud

(57) **Abstract**

The present invention relates to an intelligent subcutaneous venous access port and method for detecting biolayers using said port, wherein the port is formed by a casing (8) which is sealed in its upper part by a membrane (6) and which houses therein a reservoir where a fluid with the medicinal product for treating the patient is introduced therein, this medicinal product being directed to the areas of the body to be treated by means of a catheter, the reservoir has therein, in direct contact with the inner fluid, a biosensor for measuring physico-chemical parameters of a bacterial biolayer formed on the biosensor and the physico-chemical parameters of the fluid inside the reservoir, the port also has wireless communications means for sending the information of parameters measured by the biosensor to the outside.

## Description

### Sector of the Art

The present invention relates to the medical devices used for the predictive detections of infections, providing an intelligent subcutaneous venous access port which allows the early detection of bacteria growth which are being formed therein before they can affect the physical integrity of the patient.

### State of the Art

Subcutaneous ports are formed by a reservoir which is introduced under the patient skin and which is connected to a central vein of the organism by means of a catheter. These ports are usually used mainly in chemotherapy or oncology treatments for administrating medicinal products to different remote parts of the body, or for drawing blood samples. Solutions of ports of this type are described in the US patents 4,802,885, US 5,558,641, US 5,562,618, US 5,613,945, or US 5,743,873.

The medicinal product to be administered to the patient is introduced in the reservoir of the port by means of a syringe whereby the needle of the syringe traverses a silicone membrane sealing the reservoir. The problem resides in that during the medicinal product administration operation, pathogenic agents can be introduced into the reservoir through the effect of driving in the supply needle transversing the dermis. This can cause infections which have serious consequences for the patient. Furthermore, it is important to highlight that the patients tend to be of advanced age with concomitant pathologies and are sometimes subjected to immunosuppressive treatments, therefore the consequences of the infection can be very serious, even including death.

These infections are produced by live or inert microorganisms which grow adhered to surfaces forming extracellular structures (formed by polysaccharides, proteins and DNA) in which they are embedded. These biological microsystems is known as bacterial biolayers.

In the beginning of the biolayer creation, when the latter are recently adhered, they are more vulnerable to the action of the antibiotics and they can be fought in a rather efficient manner. The problem is that the bacteria in these initial times do not present any symptom in the patient. When the first symptoms are presented in the patient, the bacteria have been accumulated in biolayers and interact as a community, developing within the biolayer an antibiotic resistance capacity due to the extracellular structure thereof.

The diagnostic of the infectious process associated to the port is currently performed by means of culturing the silicone membrane removed from the reservoir since it is the most profitable and reliable method. To identify and confirm the caused pathogen, it is necessary to culture the sample removed from 18 to 24 hours in different specific media. Once this is done, the most effective acting drug is selected and the patient, who has had to stop the treatment that he was receiving, is treated. However, this method continues being performed after the patient presents the symptoms despite the fact that follow-up samples are taken from the content of the reservoir.

US patent 7,070,591 discloses a subcutaneous venous access port which is provided with a unit for monitoring physiological parameters of the patient, such as blood pressure, arterial pressure, blood flow, temperature, or level of gas in blood, among others. This port has the same problem as the previous solutions since physical variables of the human body are monitored and the treatment against the possible infection is only carried out once a symptom has been detected in the patient.

### Object of the Invention

According to the present invention, an intelligent subcutaneous venous access port capable of detecting the formation of bacterial biolayers therein which are the cause of possible infections that can affect the physical integrity of the patients is proposed.

The subcutaneous venous access port is formed by a casing which is sealed in its upper part by a membrane and which houses therein a reservoir where a fluid with the medicinal product for treating patient is introduced therein. The medicinal product is directed to the areas of the body to be treated by means of a catheter which is implanted in a main vein of the patient.

The reservoir of the port has therein, in direct contact with the inner fluid, a biosensor for measuring physico-chemical parameters of a bacterial biolayer formed on the biosensor and for measuring physico-chemical parameters of the fluid inside the reservoir. By using a biosensor of these characteristics the appearance of harmful microorganisms inside the reservoir can be detected before they cause an infection of serious consequence for the patient.

The biosensor used for the early detection of bacterial biolayers is an electric impedance sensor based on thin-film interdigitated microelectrodes on which the bacterial biolayers are directly formed. Nevertheless, to enable obtaining more information about the microorganisms forming the bacterial biolayer, it has been provided that the biosensor is a multiparameter sensor capable of measuring electric impedance, for detecting the bacterial biolayer formed thereon, as well as measuring the PH, the temperature, the amount of oxygen, the amount of carbon dioxide, the electric charge, or other physico-chemical parameters of the fluid inside the reservoir wherein the bacterial biolayers are formed.

In the lower part of the casing of the port there is arranged an envelope internalizing an electronic and communications plate which is responsible for processing the information measured by the biosensor. This electronic and communications plate is linked to the biosensor through a connection plate which in turn performs the isolating functions, preventing the fluid inside the reservoir from being able to penetrate inside the envelope housing the electronic and communications plate.

The subcutaneous venous access port of the present invention has wireless communications means for sending the information of physico-chemical parameters measured by the biosensor to the outside. According to a preferred embodiment, said communications means are a communications antenna. This antenna is adapted to the shape of the casing of the port, whereby providing a compact device with good technical performances and low cost is assured. The antenna can be coupled to the outside or the inside of the casing, or even embedded in the casing itself.

More specifically the communications antenna is made up of at least one interconnection path adapted to the casing and covers it partially, as well as of an upper metal ring arranged continuously to the interconnection path and adapted to the upper part of the casing. The possibility that the antenna is only made up of the interconnection path or that it is only made up of the upper metal ring has been provided. The possibility that the communications antenna has a metal base by way of ground plane in the lower part of the casing of the port has also been provided.

The interconnection path adapted to the shape of the casing of the port can have different shapes, such as for example a straight, semilunar, sinusoidal, or meandering section. Likewise, the interconnection path of the communications antenna has a width between 100 microns and 10 millimeters for all the cases with the exception of the of semilunar shape.

The invention also proposes a method for detecting bacterial biolayers by means of the intelligent subcutaneous venous access port. The method comprises the steps of:
● measuring directly inside the reservoir the electric impedance of a bacterial biolayer formed on the biosensor.
● sending the information obtained by the biosensor to an external device by means of the wireless communications means.

In addition to the measurement of electric impedance, the measurement of other physico-chemical parameters of the fluid which is inside the reservoir can be carried out, wherein that fluid relates to the biosystem wherein the bacterial biolayer can be formed. Subsequently these physico-chemical parameters of the fluid together with the electric impedance measured are sent to the external device which analyzes them and detects if a biolayer is forming inside the reservoir, as well as what are the characteristics of that bacterial biolayer which is being formed.

### Description of the Drawings

Figure 1 shows a view of a conventional subcutaneous venous access port implanted in the body of a patient.
Figure 2 shows a schematic cross-section view of a conventional subcutaneous venous access port during a medicinal product administration phase.
Figure 3 shows a perspective view of the subcutaneous venous access port of the invention.
Figure 4 is an exploded perspective view of the elements making up the subcutaneous venous access port.
Figure 5 shows a cross-section perspective view according to a vertical plane passing through the center of the subcutaneous venous access port.
Figures 6.1 to 6.6 show different variants of the communications antenna adapted to the shape of the port.

### Detailed Description of the Invention

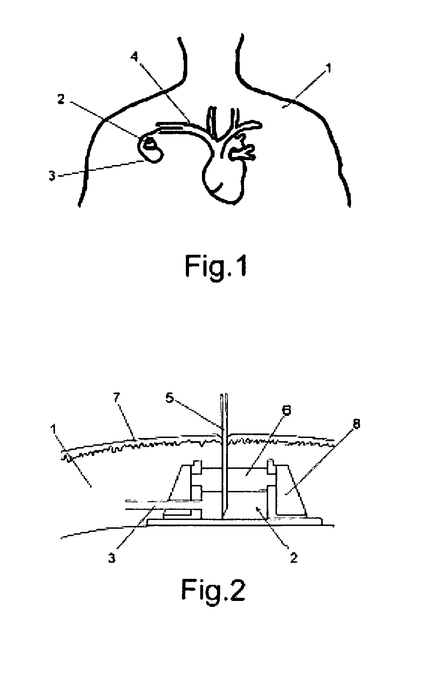

Figure 1 schematically shows a conventional subcutaneous venous access port introduced under the skin of the body (1) of the patient to be treated. The port is made up of a reservoir (2), generally from titanium with silicone, and a catheter (3) which is introduced directly in a central vein (4) of the human body, mainly the subclavian vein. To place the port, the health representative palpates the skin of the patient and by means of a syringe introduce the medicinal product in the reservoir (2) so that it is distributed towards the area of the body to be treated therefrom.

As can be observed in the schematic view of Figure 2, a silicone membrane (6) sealing the inside of the reservoir (2) where the medicinal product is introduced therein is traversed by means of a needle (5) of the syringe. When the needle (5) traverses the dermis (7), pathogenic agents are driven in such that they are introduced inside the reservoir (2), this can give rise to the formation of bacterial biolayers inside the reservoir (2) which can be the cause of an infection.

By means of the intelligent subcutaneous venous access port of the invention the conditions of the fluid which is inside the reservoir (2) are monitored. In the conventional solutions the physiological parameters of the patient are monitored, whereby any treatment is performed after an infection has been produced. The invention aims to monitor the conditions inside the reservoir (2), and, mainly the bacterial biolayer growth inside the same such that the formation of the bacterial biolayer can be fought when the latter is vulnerable to the antibiotics, thus foreseeing a possible infection before it affects the patient.

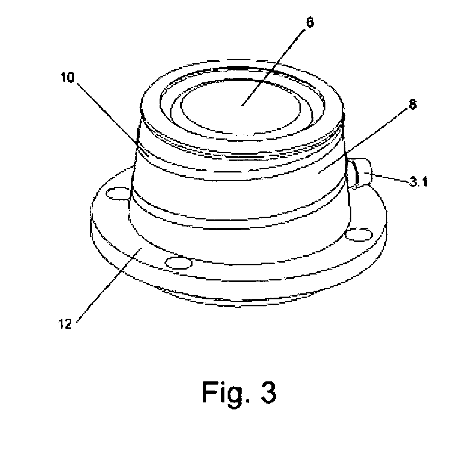

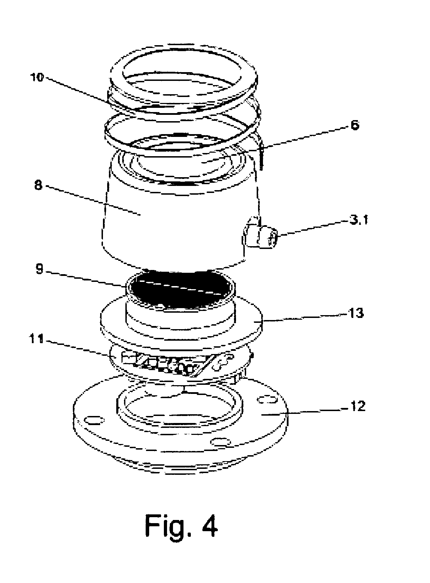

The intelligent subcutaneous venous access port object of the present invention and shown in detail in Figures 3, 4 and 5 is made up of a casing (8) of biocompatible material, with body of revolution shape, generally frustoconical shaped, and the inside of which is arranged a reservoir (2) where a solution with the medicinal product to be administered to the patient is introduced therein. The casing (8) also has an outlet (3.1) for coupling the catheter (3). The reservoir (2) is isolated from the rest of the body by its upper part by means of a silicone membrane (6) or the like, the silicone is a material which allows the reservoir (2) to remain perfectly sealed once removing the needle (5). Inside the reservoir (2), preferably in its lower part, there is arranged a biosensor (9) which is in direct contact with the fluid therein and which measures the physico-chemical parameters of said fluid. The intelligent subcutaneous port has wireless communications means for sending the parameters measured by the biosensor (9) to an external device.

According to a preferred embodiment of the invention, the wireless communications means are made up of a communications antenna (10) which is directly adapted in the casing (8) itself, this antenna (10) transmits the physico-chemical parameters of the fluid inside the reservoir (2) measured by the biosensor (9) towards an exterior receiver (not depicted). This solution is in no case limiting, another type of wireless communications means being able to exist; they can be bluetooth, infrared, etc.

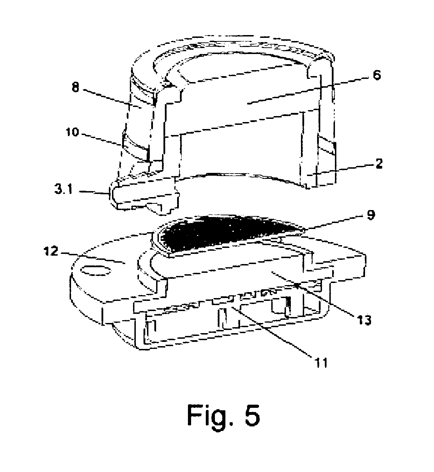

In the cross-section view of Figure 5, it can be observed that the intelligent subcutaneous venous access port has in its lower part an electronic and communications plate (11) which is isolated from the outside of the body (1) by means of a envelope (12), said electronic and communications plate (11) being linked to the biosensor (9) by means of a connection plate (13) which in turn isolates the plate (11) and from the fluid inside the reservoir (2). With this arrangement, the casing (8) is coupled above the envelope (12), the biosensor (9) being completely isolated inside the reservoir (2), to thus enable analyzing the conditions of the inner fluid and to enable predicting the formation of bacterial biolayers.

According to a preferred embodiment of the invention, the biosensor (9) is an electric impedance sensor based on thin-film microelectrodes which are in direct contact with the fluid inside the reservoir (2). When pathogenic agents are introduced inside the reservoir (2), a bacterial biolayer starts to form, this biolayer is formed on the inner surfaces of the reservoir (2) and therefore directly on the interdigitated microelectrodes of the biosensor (9), such that a change in the electric impedance which the biosensor (9) is monitoring, is sign that a bacterial biolayer is being formed inside the reservoir (2).

For detecting the bacterial biolayer, a variable electric power is introduced at different frequencies and the electric current circulating through the interdigitated microelectrodes is measured. The opposition to the passage of current (electric impedance) is very sensitive to the variations of the matter adhered to the surface of the biosensor (9), therefore, as soon as a small amount of matter is deposited on the interdigitated electrodes, a change in the electric impedance is produced which is sign of the formation of a bacterial biolayer. Thus the formation of the biolayer can be treated before an infection is produced in the patient.

Electric impedance is the optimum solution for detecting the formation of a bacterial biolayer before an infection is produced, nevertheless it has been provided that the biosensor (9) is a multiparameter sensor which besides measuring the electric impedance for detecting the bacterial biolayer, monitors other physico-chemical parameters of the fluid inside the reservoir (2) wherein that bacterial biolayer is being formed, the parameters can be, such as the PH, the temperature, the amount of oxygen, carbon dioxide, electric charge, among others. Thus, besides detecting the formation of a biolayer, information of the type of microorganisms forming them can be obtained, whereby the treatment can be more effective.

The communications antenna (10) is adapted to the shape of the casing (8) of the port and is devised for establishing the communications of the latter with a transmitter/receiver having a reading interface outside the human body. The communications antenna (10) can function in several frequency bands at the same time, it preferably functions in the 400.150-406.000 MHz bands ("MedRadio Service") of the active medical ports, and the 2.4 GHz band (ISM "Industrial, Scientific and Medical"), however with the same typology of antenna it can communicate in other frequency bands.

In the example of preferred embodiment based on an antenna (10) as wireless communications means, it has been provided that both the casing (8) and the reservoir (2) are manufactured with non-metal materials such that they do not affect the transmission efficiency of the antenna (10).

The communications antenna (10) is made up of at least one interconnection path (10.1) adapted to the casing (8) of the port partially covering its surface. Additionally, in order to improve the radiation, the communications antenna (10) can have an upper metal ring (10.2) contiguous to the upper end of the interconnection path (10.1). The upper metal ring (10.2) coincides with the upper opening defined in the casing (8) and it must have a width sufficient for leaving a free space in its center and thus enabling the insertion of the needle (5) administrating the medicinal product. The case of which the communications antenna (10) is only formed by the upper metal ring (10.2) can also be a possibility. On the other hand, the communications antenna (10) has the possibility of having a metal base (10.3) by way of ground plane in the lower part of the casing (8).

It has been provided that the communications antenna (10) is adapted to the outer surface of the casing (8) of the port, but nevertheless it can also go through the inside of the casing (8), or be embedded in the casing (8) itself without significantly altering the characteristics of the communications. It is evident that the casing (8) and the reservoir (2) can be made up of a single piece without it altering the concept of the invention. In this case the communications antenna (10) can be adapted to the outside of that single piece, or embedded therein, but it could not be adapted to the inside of that piece, since in that case the antenna (10) would be in contact with the inner fluid.

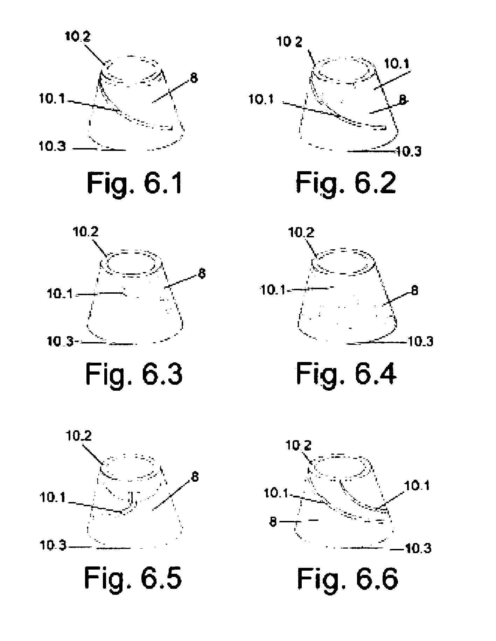

As shown in Figures 6.1 to 6.6 the communications antenna (10) can adopt different configurations. Thus, the interconnection paths (10.1) which are adapted to the shape of the casing (8) can be straight sections (Figures 6.1, 6.2, 6.5 and 6.6), semilunar sections (Figure 6.2 and 6.5), sinusoidal section (Figure 6.3), or meandering section (figure 6.4). It has also been provided that, to assure a good communications of the port within the pre-established frequency bands, the interconnection path (10.1) of the communications antenna (10) has a width between 100 microns and 10 millimeters, this is achieved in all the cases with the exception of the semilunar shape.

The upper metal ring (10.2) can have different configurations, being able to be continuous, discontinuous, of constant width or variable width. As observed in Figure 6.6, the interconnection path (10.1) can end in an open circuit or it can end in a short circuit with the metal base (10.3). Likewise in said figure the possibility of having a wide variety of interconnection paths (10.1) adapted to casing (8) of the port can be observed. To supply power to the communications antenna (10), a single power point on the interconnection paths (10.1) or the upper metal ring (10.2), or several strategically distributed power points can be used.

## Claims

1. An intelligent subcutaneous venous access port formed by a casing (8) which is sealed in its upper part by a membrane (6) and which houses therein a reservoir (2) where a fluid with the medicinal product for treating the patient is introduced therein, this medicinal product being directed to the areas of the body (1) to be treated by means of a catheter (3), **characterized in that** the reservoir (2) has therein, in direct contact with the inner fluid, a biosensor (9) for measuring physico-chemical parameters of a bacterial biolayer formed on the biosensor (9) and physico-chemical parameters of the fluid inside the reservoir (2); and **in that** it has wireless communications means for sending the information of parameters measured by the biosensor (9) to the outside.

2. The intelligent subcutaneous venous access port according to claim 1, **characterized in that** the biosensor (9) is an electric impedance sensor based on thin-film interdigitated microelectrodes on which the bacterial biolayer is directly formed.

3. The intelligent subcutaneous venous access port according to claim 1, **characterized in that** the biosensor (9) is a multiparameter sensor on which the bacterial biolayer is formed and which measures physico-chemical parameters of said bacterial biolayer and of the fluid inside the reservoir (2) where the bacterial biolayer is.

4. The intelligent subcutaneous venous access port according to claim 1, **characterized in that** coupled to the lower part of the casing (8) there is an envelope (12) internalizing an electronic and communications plate (11) processing the information measured by the biosensor (9), said electronic and communications plate (11) being linked to the biosensor (9) through a connection plate (11) which performs isolating functions.

5. The intelligent subcutaneous venous access port according to claim 1, **characterized in that** the wireless communications means are a communications antenna (10) which is arranged in the port itself adapted to the shape of the casing (8).

6. The intelligent subcutaneous venous access port according to claim 5, **characterized in that** the communications antenna (10) is made up of at least one interconnection path (10.1) adapted to the casing (8) and covers it partially, and of an upper metal ring (10.2) arranged continuously to the interconnection path and adapted to the upper part of the casing (8).

7. The intelligent subcutaneous venous access port according to claim 5, **characterized in that** the communications antenna (10) is made up of at least one interconnection path (10.1) adapted to the casing (8) and covers it partially.

8. The intelligent subcutaneous venous access port according to claim 5, **characterized in that** the communications antenna (10) is made up of an upper metal ring (10.2) adapted to the upper part of the casing (8).

9. The intelligent subcutaneous venous access port according to claim 5, **characterized in that** the communications antenna (10) has a metal base (10.3) by way of ground plane in the lower part of the casing (8).

10. The intelligent subcutaneous venous access port according to claims 5, 6, 7, 8 and 9, **characterized in that** the communications antenna (10) is arranged coupled to the outside or the inside of the casing (8), or embedded in the casing (8) itself.

11. The intelligent subcutaneous venous access port according to claims 6 and 7, **characterized in that** the interconnection path (10.1) of the communications antenna (10) has a width between 100 microns and 10 millimeters.

12. The intelligent subcutaneous venous access port according to claims 6 and 7, **characterized in that** the interconnection path (10.1) adapted to the shape of the casing (8) is a straight, semilunar, sinusoidal, or meandering section.

13. A method for detecting biolayers by means of the intelligent subcutaneous venous access port of the preceding claims, **characterized in that** it comprises the steps of:
● measuring inside a reservoir (2) the electric impedance of a bacterial biolayer formed on a biosensor (9).
● sending the information obtained by the biosensor (9)by means of wireless communications means to an external device.

14. The method for detecting biolayers according to the preceding claim, **characterized in that** the physico-chemical parameters of the fluid inside the reservoir (2) wherein the bacterial biolayer is being formed are measured by means of the biosensor (9), and these parameters are sent to an external device by means of the wireless communications means.
